# EUROPÄISCHE PATENTSCHRIFT

(11) **EP 1 087 975 B1**
(45) Veröffentlichungstag und Bekanntmachung des Hinweises auf die Patenterteilung: **27.08.2003**
(21) Anmeldenummer: 99932700.0
(22) Anmeldetag: 18.06.1999
(51) Int. Cl.: C07D 493/04, C07D 417/06, C07D 413/06, C07D 277/24

(54) **EPOTHILON-NEBENKOMPONENTEN**
EPOTHILONE MINOR CONSTITUENTS
CONSTITUANTS SECONDAIRES D'EPOTHILONE

(30) Priorität: 18.06.1998 DE 19826988
(43) Veröffentlichungstag der Anmeldung: 04.04.2001
(60) Teilanmeldung: 02022332.7
(73) Patentinhaber: Gesellschaft für Biotechnologische Forschung mbH (GBF), 38124 Braunschweig (DE)
(72) Erfinder: HOEFLE, Gerhard, D-38124 Braunschweig (DE); REICHENBACH, Hans, D-38124 Braunschweig (DE); GERTH, Klaus, D-38124 Braunschweig (DE); HARDT, Ingo, D-38124 Braunschweig (DE); SASSE, Florenz, D-38124 Braunschweig (DE); STEINMETZ, Heinrich, D-38124 Braunschweig (DE)
(74) Vertreter: Boeters, Hans Dietrich, Dr.
(86) Internationale Anmeldenummer: EP9904244
(87) Internationale Veröffentlichungsnummer: WO99065913

(56) Entgegenhaltungen:
- WO-A-97/19086
- WO-A-98/08849
- WO-A-98/22461
- NICOLAOU K C ET AL: "DESIGNED EPOTHILONES: COMBINATORIAL SYNTHESIS, TUBULIN ASSEMBLY PROPERTIES, AND CYTOTOXIC ACTION AGAINST TAXOL.RESISTANT TUMOR CELLS" ANGEWANDTE CHEMIE. INTERNATIONAL EDITION,DE,VERLAG CHEMIE. WEINHEIM, Bd. 36, Nr. 19, 1. Januar 1997 (1997-01-01), Seiten 2097-2103, XP002064441 ISSN: 0570-0833
- NICOLAOU ET AL: "Total synthesis of oxazole- and cyclopropane-containing epothilone A analogs by the olefin metathesis approach" CHEMISTRY - A EUROPEAN JOURNAL,US,VCH PUBLISHERS, Bd. 3, Nr. 12, 1997, Seiten 1957-1970, XP002121565 ISSN: 0947-6539
- BALOG A ET AL: "Stereoselective Syntheses and Evaluation of Compounds in the 8-Desmethylepothilone A Series: Some Surprising Observations Regarding Their Chemical and Biological Properties" TETRAHEDRON LETTERS,NL,ELSEVIER SCIENCE PUBLISHERS, AMSTERDAM, Bd. 38, Nr. 26, 30. Juni 1997 (1997-06-30), Seiten 4529-4532, XP004074826 ISSN: 0040-4039
- SU D -S ET AL: "STRUCTURE - ACTIVITY RELATIONSHIPS OF THE EPOTHILONES AND THE FIRST IN VIVO COMPARISON WITH PACLITAXEL" ANGEWANDTE CHEMIE. INTERNATIONAL EDITION,DE,VERLAG CHEMIE. WEINHEIM, Bd. 36, Nr. 19, 1997, Seiten 2093-2096, XP002916075 ISSN: 0570-0833
- K.C. NICOLAOU ET AL.: "Probing the ring size of epothilones: total synthesis of [14]-, [15]-, [17]-, and [18]Epothilones A" ANGEWANDTE CHEMIE INTERNATIONAL EDITION., Bd. 37, Nr. 1/2, 1998, Seiten 81-84, XP002131226 WEINHEIM DE

## Beschreibung

Die Erfindung betrifft Verbindungen, die im vorliegenden Zusammenhang als Epothilon-Nebenkomponenten bezeichnet werden, und zwar Verbindungen 5 bis 7, 16 bis 19, 26 und 28 bis 29. Diese Verbindungen lassen sich durch Fermentation von DSM 6773 gemäß DE 41 38 042.8 gewinnen.

Kenndaten der erfindungsgemäßen Verbindungen sind im folgenden zusammengestellt.

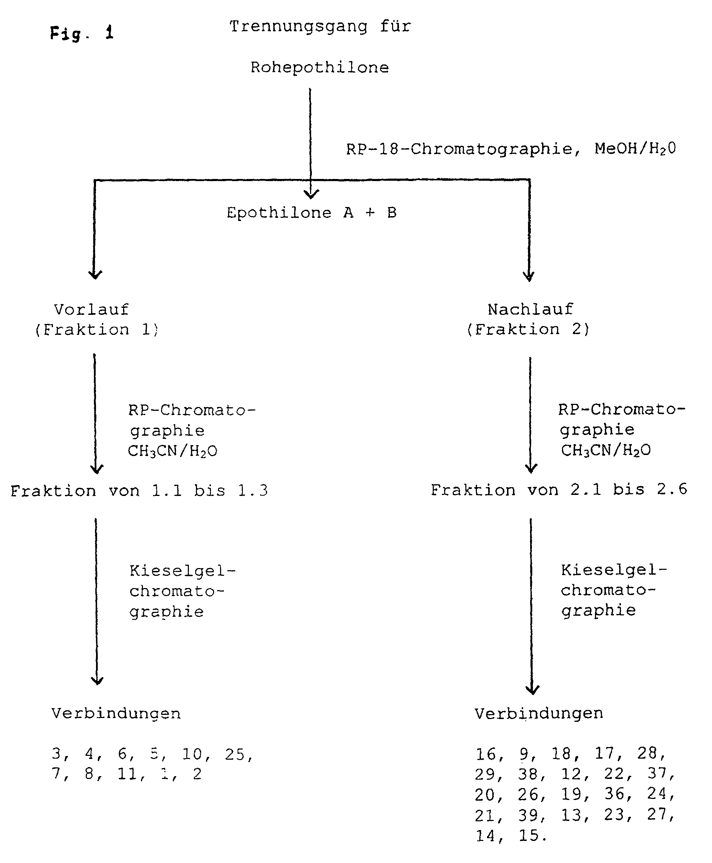

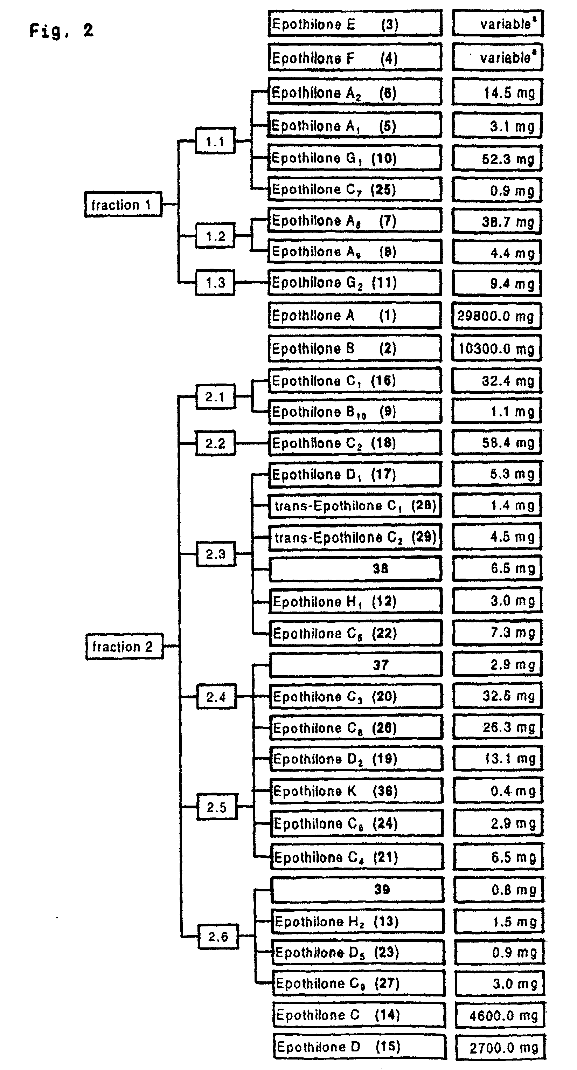

Gewinnung: Die Aufarbeitung eines Rohepothilon-Gemischs, das durch Fermentation von DSM 6773 in einem 900 Liter-Fermentator gewonnen wurde, ist schematisch Fig. 1 bis 2 zu entnehmen.

Aktivitäten: vgl. Tab. 1

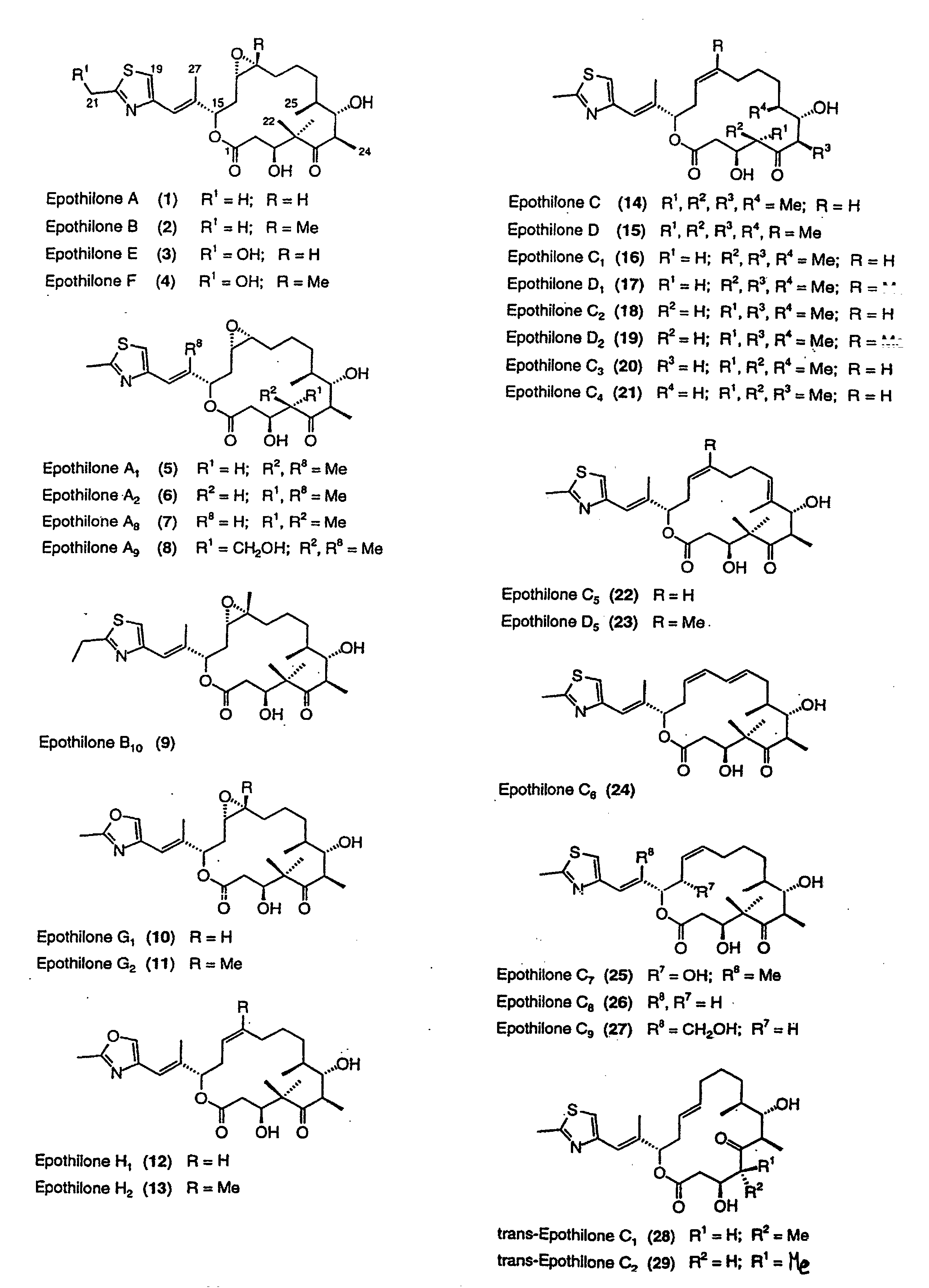

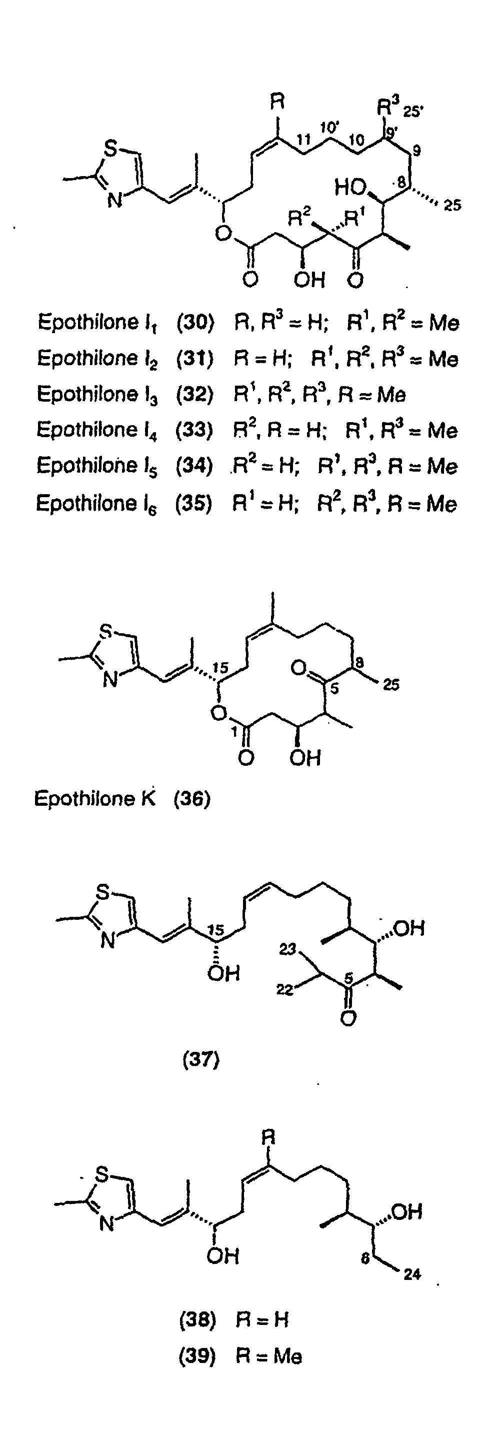

**Epothilone A**_{**1**} **(5):** colorless amorphous solid; [α]²²_{D} -69 (*c* 0.1, MeOH); UV (MeOH) λₘₐₓ nm (ε) 208 (19600), 247 (13600); IR (KBr) νₘₐₓ 3437, 2959, 2931, 2876, 1732, 1710, 1455, 1259, 978 cm⁻¹; ¹H NMR (CDCl₃, 400 MHz) δ 6.95 (1H, s, H-19), 6.60 (1H, bs, H-17), 5.68 (1H, dd, *J=* 4.4, 4.0 Hz, H-15), 4.12 (1H, m, H-3), 3.71 (1H, m, H-7), 3.52 (1H, bs, 7-OH), 3.37 (1H, bd, *J=* 7.5 Hz, 3-OH), 3.21 (1H, dq, *J =* 7.7, 7.0 Hz, H-4), 3.02 (1H, ddd, *J* = 9.2, 4.5, 2.8 Hz, H-13), 2.87 (1H, ddd, *J* = 8.3, 4.5, 3.7 Hz, H-12), 2.78 (1H, dd, *J* = 16.8, 4.3 Hz, H-2a), 2.70 (3H, s, H-21), 2.66 (1H, dq, *J=* 3.9, 7.0 Hz, H-6), 2.65 (1H, dd, *J* = 16.8, 5.2 Hz, H-2b), 2.16 (1H, ddd, *J=* 15.4, 4.4, 2.8 Hz, H-14a), 2.12 (3H, bs, H-27), 1.91 (1H, ddd, *J=* 15.4, 9.2, 4.0 Hz, H-14b), 1.63 (1H, m, H-10a), 1.62 (2H, m, H-11), 1.59 (1H, m, H-9a), 1.52 (1H, m, H-10b), 1.39 (1H, m, H-8), 1.35 (1H, m, H-9b), 1.211 (3H, d, *J*= 7.0 Hz, H-23), 1.207 (3H, d, *J* = 7.0 Hz, H-24), 0.89 (3H, d, *J*= 6.9 Hz, H-25); EIMS *m*/*z* 479 [M]⁺ (21), 322 (31), 306 (65), 304 (47), 168 (45), 166 (73), 164 (100), 151 (30). 140 (35); HREIMS *m*/*z* 479.2317 (calcd. for C₂₇H₄₁NO₅S, 479.2342).
**Epothilone A**_{**2**} **(6):** colorless amorphous solid; [α]²²_{D} +12.0 (*c* 1.0, MeOH); UV (MeOH) λₘₐₓ nm (ε) 210 (15100), 248 (15500); IR (KBr) νₘₐₓ 3438, 2963, 2929, 2875, 1734, 1706, 1458, 1262, 981 cm⁻¹; ¹H NMR (CDCl₃, 400 MHz) δ 6.98 (1H, s, H-19), 6.63 (1H, bs, H-17), 5.40 (1H, dd, *J* = 8.3, 3.4 Hz, H-15), 4.26 (1H, ddd, *J=* 8.5, 4.8, 4.7 Hz, H-3), 3.85 (1H, dd, *J*= 7.9, 2.6 Hz, H-7), 3.54 (1H, bs, 3-OH), 3.09 (1H, dq, *J*= 4.8, 7.0 Hz, H-4), 3.01 (1H, ddd, *J* = 8.3, 4.8, 4.6 Hz, H-13), 2.98 (1H, dq, *J* = 7.9, 7.0 Hz, H-6), 2.89 (1H, ddd, *J*= 6.7, 4.6, 4.4 Hz, H-12), 2.68 (3H, s, H-21), 2.60 (1H, dd, *J* = 15.1, 8.5 Hz, H-2a), 2.52 (1H, bs, 7-OH), 2.50 (1H, dd, *J* = 15.1, 4.7 Hz, H-2b), 2.18 (1H, ddd, *J* = 15.0, 4.8, 3.4 Hz, H-14a), 2.11 (3H, d, *J* = 1.3 Hz, H-27), 1.82 (1H, ddd, *J* = 15.0, 8.3, 8.1 Hz, H-14b), 1.63 (1H, m, H-8), 1.61 (2H, m, H-11a and H-10a), 1.46(1H, m, H-11b), 1.39 (2H, m, H-9), 1.31 (1H, m, H-10b), 1.22 (3H, *d*, *J* = 7.0 Hz, H-24), 1.15 (3H, d, *J* = 7.0 Hz, H-22), 1.01 (3H, d, *J* = 6.9 Hz, H-25); ¹³C NMR (CDCl₃, 100 MHz) δ 216.2 (s, C-5), 170.1 (s, C-1), 164.9 (s, C-20), 152.0 (s, C-18), 137.0 (s, C-16), 120.3 (d, C-17), 116.5 (d, C-19), 76.7 (d, C-15), 75.6 (d, C-7), 69.1 (d, C-3), 57.1 (d, C-12), 54.3 (d, C-13), 50.3 (d, C-4), 49.6 (d, C-6), 39.4 (t, C-2), 35.5 (d, C-8), 32.2 (t, C-14), 29.6 (t, C-9), 27.6 (t, C-11), 23.9 (t, C-10), 19.2 (q, C-21), 18.0 (q, C-25), 15.6 (q, C-27), 13.9 (q, C-24), 12.4 (q, C-22); EIMS *m*/*z* 479 [M]⁺ (18), 322 (38), 306 (78), 304 (59), 168 (48), 166 (96), 164 (100), 151 (33), 140 (38); HREIMS *m*/*z* 479.2318 (calcd. for C₂₇H₄₁NO₅S, 479.2342).
**Epothilone A**_{**8**} **(7):** colorless amorphous solid; [α]²²_{D} -76.2 (*c* 1.0, MeOH); UV (MeOH) λₘₐₓ nm (ε) 210 (15300), 248 (15500); IR (KBr) νₘₐₓ 3440, 2967, 2932, 2876, 1736, 1691, 1467, 1252, 979 cm⁻¹; ¹H NMR (CDCl₃, 400 MHz) δ 6.95 (1H, s, H-19), 6.64 (1H, dd, *J* = 15.6, 0.9 Hz, H-17), 6.52 (1H, dd, *J* = 15.6, 6.6 Hz, H-16), 5.68 (1H, dddd, *J* = 7.8, 6.6, 3.2, 0.9 Hz, H-15), 4.11 (1H, ddd, *J* = 10.1, 6.6, 3.5 Hz, H-3), 3.78 (1H, ddd, *J* = 5.2, 3.2, 3.2 Hz, H-7), 3.66 (1H, d, *J* = 6.6 Hz, 3-OH), 3.23 (1H, dq, *J* = 5.2, 6.9 Hz, H-6), 3.08 (1H, ddd, *J* = 7.3, 5.5,4.1 Hz, H-13), 2.90 (1H, ddd, *J* = 6.6, 4.6, 4.1 Hz, H-12), 2.69 (3H, s, H-21), 2.52 (1H, dd, *J* = 14.7, 10.1 Hz, H-2a), 2.44 (1H, bd, *J* = 3.2 Hz, 7-OH), 2.41 (1H, dd, *J* = 14.7, 3.5 Hz, H-2b), 2.10 (1H, ddd, *J* = 15.0, 5.5, 3.2 Hz, H-14a), 1.90 (1H, ddd, *J* = 15.0, 7.8, 7.3 Hz, H-14b), 1.71 (1H, m, H-8), 1.65 (1H, m, H-11a), 1.50 (1H, m, H-10a), 1.47 (1H, m, H-11b), 1.40 (2H, m, H-9), 1.39 (1H, m, H-10b), 1.33 (3H, s, H-23), 1.16 (3H, d, *J* = 6.9 Hz, H-24), 1.08 (3H, s, H-22), 0.98 (3H, d, *J* = 7.0 Hz, H-25); ¹³C NMR (CDCl₃, 75 MHz) δ 220.3 (s, C-5), 170.7 (s, C-1), 166.5 (s, C-20), 152.2 (s, C-18), 128.4 (d, C-16), 125.9 (d, C-17), 116.4 (d, C-19), 75.0 (d, C-7), 73.6 (d, C-3), 72.7 (d, C-15), 57.3 (d, C-12), 54.1 (d, C-13), 52.6 (s, C-4), 43.8 (d, C-6), 38.9 (t, C-2), 36.3 (d, C-8), 32.5 (t, C-14), 30.3 (t, C-9), 26.7 (t, C-11), 24.0 (t, C-10), 21.3 (q, C-23), 21.0 (q, C-22), 19.3 (q, C-21), 17.1 (q, C-25), 14.5 (q, C-24); EIMS *m*/*z* 479 [M]⁺ ; HRDCIMS *m*/*z* 480.2401 (calcd. for C₂₅H₃₈NO₆S, 480.2401).
**Epothilone C**_{**1**} **(16):** colorless amorphous solid; [α]²²_{D} -114.0 (*c* 10.0, MeOH); UV (MeOH) λₘₐₓ nm (ε) 211 (16500), 248 (12500); IR (KBr) νₘₐₓ 3440, 2933, 2877, 2858, 1730, 1708, 1457, 1244, 981 cm⁻¹; ¹H NMR (CDCl₃, 300 MHz) δ 6.96 (1H, s, H-19), 6.56 (1H, bs, H-17), 5.47 (1H, dd, *J* = 9.2, 3.0 Hz, H-15), 5.43 (1H, m, H-12), 5.40 (1H, m, H-13), 4.40 (1H, ddd, *J* = 6.2, 6.1, 6.1 Hz, H-3), 3.69 (1H, dd, *J =* 5.7, 3.6 Hz, H-7), 3.01 (1H, dq, *J* = 5.7, 6.9 Hz, H-6), 3.01 (1H, bs, 3-OH), 2.84 (1H, dq, *J =* 5.2, 7.0 Hz, H-4), 2.68 (3H, s, H-21), 2.66 (1H, ddd, *J* = 16.4, 9.2, 7.3 Hz, H-14a), 2.64 (1H, dd, *J* = 15.9, 7.1 Hz, H-2a), 2.54 (1H, dd, *J* = 15.9, 6.1 Hz, H-2b), 2.38 (1H, bd, *J* = 16.4 Hz, H-14b), 2.35 (1H, bs, 7-OH), 2.07 (3H, bs, H-27), 2.03 (2H, m, H-11), 1.62 (1H, m, H-10a), 1.53 (1H, m, H-8), 1.35 (1H, m, H-9a), 1.22 (1H, m, H-9b), 1.19 (3H, d, *J =* 6.9 Hz, H-24), 1.14 (3H, d, *J* = 6.9 Hz, H-23), 1.10 (1H, m, H-10b), 0.95 (3H, d, *J* = 6.9 Hz, H-25); ¹³C NMR, see Table 1; EIMS *m*/*z* 463 [M]⁺ (5), 324 (8), 290 (8), 204 (7), 168 (100), 164 (15), 139 (36); HREIMS *m*/*z* 463.2381 (calcd. for C₂₅H₃₇NO₅S, 463.2392).
**Epothilone D**_{**1**} **(17):** colorless amorphous solid; [α]²²_{D} -118.6 (*c* 0.5, MeOH); UV (MeOH) λₘₐₓ nm (ε) 208 (18300), 249 (11900); IR (KBr) νₘₐₓ 3439, 2965, 2934, 2877, 1729, 1707, 1456, 1250, 980 cm⁻¹; ¹H NMR (CDCl₃, 300 MHz) δ 6.98 (1H, s, H-19), 6.56 (1H, bs, H-17), 5.51 (1H, dd, *J* = 9.5, 3.4 Hz, H-15), 5.16 (1H, dd, *J* = 8.0, 4.2 Hz, H-13), 4.42 (1H, ddd, *J* = 7.1, 6.3, 5.5 Hz, H-3), 3.70 (1H, dd, *J* = 6.5, 2.9 Hz, H-7), 3.07 (1H, dq, *J* = 6.5, 6.9 Hz, H-6), 2.95 (1H, dq, *J* = 4.7, 7.0 Hz, H-4), 2.71 (3H, s, H-21), 2.69 (1H, dd, *J* = 16.0, 6.3 Hz, H-2a), 2.64 (1H, m, H-14a), 2.59 (1H, dd, *J* = 16.0, 7.1 Hz, H-2b), 2.46 (1H, bs, 3-OH), 2.38 (1H, bd, *J*= 16.0 Hz, H-14b), 2.19 (1H, ddd, *J* = 13.3, 8.6, 5.7 Hz, H-11a), 2.10 (3H, d, *J* = 1.4 Hz, H-27), 2.02 (1H, bs, 7-OH), 1.91 (1H, ddd, *J* = 13.3, 6.0, 6.0 Hz, H-11b), 1.68 (1H, m, H-10a), 1.66 (3H, bs, H-26), 1.53 (1H, m, H-8), 1.37 (1H, m, H-9a), 1.26 (1H, m, H-9b), 1.24 (3H, d, *J* = 6.9 Hz, H-24), 1.19 (1H, m, H-10b), 1.14 (3H, d. *J =* 7.0, H-23), 0.99 (3H, d, *J =* 6.9 Hz, H-25); ¹³C NMR (CDCl₃, 100 MHz) δ 217.0 (s, C-5), 169.7 (s, C-1), 165.0 (s, C-20), 152.2 (s, C-18), 138.5 (s, C-12), 137.7 (s, C-16), 120.7 (d, C-13), 120.1 (d, C-17), 116.3 (d, C-19), 78.8 (d, C-15), 77.2 (d, C-7), 67.7 (d, C-3), 52.1 (d, C-4), 46.5 (d, C-6), 40.6 (t, C-2), 37.6 (d, C-8), 32.3 (t, C-14), 31.8 (t, C-11), 29.5(t, C-9), 25.5 (t, C-10), 23.1 (q, C-26), 19.2 (q, C-21), 15.5 (q, C-27), 16.6 (q, C-25), 14.5 (q, C-24), 9.7 (q, C-23); EIMS *m*/*z* 477 [M]⁺ (13), 304 (19), 303 (31), 218 (40), 204 (41), 168 (100), 164 (45), 157 (25), 139 (18); HREIMS *m*/*z* 477.2544 (calcd. for C₂₆H₃₉NO₅S, 477.2549).
**Epothilone C**_{**2**} **(18):** colorless amorphous solid; [α]²²_{D} -11.6 (*c* 10.0, MeOH); UV (MeOH) λₘₐₓ nm (ε) 212 (15500), 249 (12100); IR (KBr) νₘₐₓ 3428, 2962, 2929, 2877, 2859, 1734, 1705, 1460, 1251, 982 cm⁻¹; ¹H NMR (CDCl₃, 300 MHz) δ 6.99 (1H, s, H-19), 6.66 (1H, bs, H-17), 5.55 (1H, ddd, *J* = 10.4, 9.2, 6.1 Hz, H-12), 5.38 (1H, ddd, *J* = 10.4, 9.3, 6.2 Hz, H-13), 5.22 (1H, dd, *J* = 8.8, 2.8 Hz, H-15), 4.42 (1H, dddd, *J* = 9.4, 5.6, 4.2, 4.1 Hz, H-3), 3.93 (1H, d, *J* = 5.6 Hz, 3-OH), 3.86 (1H, m, H-7), 3.15 (1H, bs, 7-OH), 3.12 (1H, dq, *J* = 4.2, 7.0 Hz, H-4), 3.00 (1H, dq, *J* = 6.9, 7.0 Hz, H-6), 2.70 (3H, s, H-21), 2.62 (1H, dddd, *J* = 15.1, 9.3, 8.8, 0.8 Hz, H-14a), 2.58 (1H, dd, *J* = 15.4, 9.4 Hz, H-2a), 2.38 (1H, dd, *J* = 15.4, 4.1 Hz, H-2b), 2.31 (1H, ddd, *J* = 15.1, 6.2, 2.8 Hz, H-14b), 2.08 (3H, d, *J* = 1.3 Hz, H-27), 2.15 (1H, m, H-11a), 2.04 (1H, m, H-11b), 1.71 (1H, m, H-10a), 1.43 (1H, m, H-9a), 1.31 (1H, m, H-9b), 1.26 (3H, d, *J* = 7.0 Hz, H-24), 1.15 (3H, d, *J* = 7.0 Hz, H-23), 1.11 (1H, m, H-10b), 1.00 (3H, d, *J* = 6.9 Hz, H-25); ¹³C NMR, see Table 1; EIMS *m*/*z* 463 [M]⁺ (7), 324 (7), 306 (8), 290 (17), 168 (100), 164 (14), 139 (27); HREIMS *m*/*z* 463.2392 (calcd. for C₂₅H₃₇NO₅S, 463.2392).
**Epothilone D**_{**2**} **(19):** colorless amorphous solid; [α]²²_{D}-12.5 (*c* 1.0, MeOH); UV (MeOH) λₘₐₓ nm (ε) 210 (15400), 248 (11200); IR(KBr) νₘₐₓ 3436, 2965, 2930, 2877, 1732, 1705, 1458, 1253, 980 cm⁻¹; ¹H NMR (CDCl₃, 400 MHz) δ 6.97 (1H, s, H-19), 6.56 (1H, bs, H-17), 5.18 (1H, dd, *J=* 7.9, 4.9 Hz, H-15), 5.18 (1H, ddd, *J =* 9.6, 5.4, 1.0 Hz, H-13), 4.27 (1H, m, H-3), 3.88 (1H, dd, *J* = 5.6, 4.6 Hz, H-7), 3.19 (1H, bs, 3-OH), 3.07 (1H, dq, *J* = 4.3, 7.0 Hz, H-4), 2.95 (1H, dq, *J* = 5.6, 7.0 Hz, H-6), 2.70 (3H, s, H-21), 2.62 (1H, dd, *J =* 14.9, 7.8 Hz, H-2a), 2.56 (1H, ddd, *J* = 14.7, 9.6, 7.9 Hz, H-14a), 2.43 (1H, dd, *J* = 14.9, 5.6 Hz, H-2b), 2.38 (1H, bs, 7-OH), 2.26 (1H, ddd. *J* = 14.5, 5.4, 4.9 Hz, H-14b), 2.19 (1H, ddd, *J =* 13.0, 10.4, 5.4 Hz, H-11a), 2.10 (3H, d, *J* = 1.4 Hz, H-27), 1.95 (1H, ddd, *J* = 13.0, 10.3, 5.3 Hz, H-11b), 1.72 (1H, m, H-8), 1.68 (3H, bs, H-26), 1.61 (1H, m, H-10a), 1.39 (2H, m, H-9), 1.21 (1H, m, H-10b), 1.19 (3H, d, *J =* 6.9 Hz, H-24), 1.17 (3H, d. *J* = 7.0, H-22), 1.00 (3H, d, *J* = 6.9 Hz, H-25); ¹³C NMR (CDCl₃, 100 MHz) δ 216.8 (s, C-5), 170.4 (s, C-1), 164.9 (s, C-20), 152.3 (s, C-18), 139.8 (s, C-12), 137.5 (s, C-16), 120.5 (d, C-17), 119.2 (d, C-13), 116.3 (d, C-19), 80.0 (d, C-15), 74.3 (d, C-7), 69.7 (d, C-3), 48.6 (d, C-4), 48.4 (d, C-6), 39.9 (t, C-2), 36.6 (d, C-8), 32.2 (t, C-14), 32.7 (t, C-11), 30.9 (t, C-9), 26.0 (t, C-10), 23.6 (q, C-26), 19.2 (q, C-21), 15.4 (q, C-27), 17.1 (q, C-25), 12.4 (q, C-24),12.7 (q, C-23); EIMS *m*/*z* 477 [M]⁺ (22), 304 (19), 303 (17), 218 (22), 204 (25), 168 (100), 164 (28), 157 (31), 139 (21); HREIMS *m*/*z* 477.2545 (calcd. for C₂₆H₃₉NO₅S, 477.2549).
**Epothilone C**_{**8**} **(26)**: colorless amorphous solid; [α]²²_{D}-75.2 (*c* 2.5, MeOH]; UV (MeOH) λₘₐₓ nm (ε) 210 (16800), 248 (17800); IR (KBr) νₘₐₓ 3443, 2932, 2881, 1734, 1689, 1465, 1255, 1183, 976 cm⁻¹; ¹H NMR (CDCl₃, 300 MHz) δ 6.93 (1H, s, H-19), 6.62 (1H, dd, *J* = 15.6, 0.6 Hz, H-17), 6.49 (1H, dd, *J =* 15.6, 6.6 Hz, H-16), 5.52 (1H, dddd, *J* = 9.5, 6.6, 2.8, 0.6 Hz, H-15), 5.42 (1H, m, H-12), 5.41 (1H, m, H-13), 4.13 (1H, ddd, *J* = 11.0, 5.3, 2.8 Hz, H-3), 3.69 (1H, ddd, *J* = 3.7, 2.8, 2.5 Hz, H-7), 3.11 (1H, dq, *J* = 2.5, 6.8 Hz, H-6), 2.95 (1H, d, *J* = 5.3 Hz, 3-OH), 2.90 (1H, d, *J* = 2.8 Hz, 7-OH), 2.69 (3H, s, H-21), 2.67 (1H, ddd, *J =* 14.9, 9.5, 8.4 Hz, H-14a), 2.48 (1H, dd, *J* = 15.6, 11.0 Hz, H-2a), 2.33 (1H, dd, *J* = 15.6, 2.8 Hz, H-2b), 2.30 (1H, bd, *J* = 14.9 Hz, H-14b), 2.14 (1H, m, H-11a), 2.03 (1H, m, H-11b), 1.71 (1H, m, H-8), 1.63 (1H, m, H-10a), 1.31 (1H, m, H-9a), 1.29 (3H, s, H-23), 1.17 (3H, d, *J* = 6.8 Hz, H-24), 1.16 (1H, m, H-10b), 1.14 (1H, m, H-9b), 1.05 (3H, s, H-22), 0.97 (3H, d, *J* = 7.1 Hz, H-25); ¹³C NMR, see Table 1; EIMS *m*/*z* 463 [M]⁺ (21), 310 (10), 276 (21), 171 (83), 154 (100), 150 (27), 111 (18); HREIMS *m*/*z* 463.2382 (calcd. for C₂₅H₃₇NO₅S, 463.2392).
**trans-Epothilone C**_{**1**} **(28)**: colorless amorphous solid; [α]²²_{D} -84 (c 0.2, MeOH); UV (MeOH) λₘₐₓ nm (ε) 211 (17400), 248 (12900); IR (KBr) νₘₐₓ 3433, 2961, 2933, 2879, 1730, 1708, 1457, 1251, 975 cm⁻¹; ¹H NMR (CDCl₃, 600 MHz) δ 7.00 (1H, s, H-19), 6.64 (1H, bs, H-17), 5.45 (1H, ddd, *J* = 15.2, 6.5, 6.5 Hz, H-12), 5.42 (1H, dd, *J* = 6.4, 3.7 Hz, H-15), 5.35 (1H, dt, *J =* 15.2, 7.1 Hz, H-13), 4.42 (1H, m, H-3), 3.58 (1H, ddd, *J* = 8.1, 7.9, 2.8 Hz, H-7), 3.24 (1H, m, H-6), 3.14 (1H, dq, *J* = 4.0, 6.9 Hz, H-6), 2.92 (1H, d, *J* = 7.9 Hz, 7-OH), 2.71 (3H, s, H-21), 2.71 (2H, m, H-2), 2.53 (2H, m, H-14), 2.17 (1H, d, *J* = 2.17 Hz, 3-OH), 2.11 (1H, m, H-11a), 2.06 (3H, bs, H-27), 1.93 (1H, m, H-11b), 1.68 (1H, m, H-9a), 1.65 (1H, m, H-10a), 1.33 (1H, m, H-8), 1.26 (3H, d, *J* = 6.8 Hz, H-24), 1.16 (1H, m, H-10b), 1.12 (3H, d, *J* = 6.9 Hz, H-22), 1.07 (1H, m, H-9b), 1.00 (3H, d, *J* = 6.8 Hz, H-25); ¹³C NMR, see Table 1; EIMS *m*/*z* 463 [M]⁺ (6), 290 (21), 289 (20), 204 (23), 194 (19), 190 (22), 168 (100), 164 (48), 157 (14), 152 (19), 151 (17), 139 (15), 111 (18); HREIMS *m*/*z* 463.2371 (calcd. for C₂₅H₃₇NO₅S, 463.2392).
**trans-Epothilone C**_{**2**} **(29):** colorless amorphous solid; [α]²²_{D} -3 (*c* 1.5, MeOH); UV (MeOH) λₘₐₓ nm (ε) 211 (15800), 248 (11900); IR (KBr) νₘₐₓ 3435, 2963, 2931, 2878, 1731, 1706, 1457, 1273, 979 cm⁻¹; ¹H NMR (CDCl₃, 600 MHz) δ 6.99 (1H, s, H-19), 6.57 (1H, bs, H-17), 5.56 (1H, ddd, *J* = 15.1, 7.4, 7.0 Hz, H-12), 5.41 (1H, ddd, *J* = 15.1, 7.0, 6.9 Hz, H-13), 5.41 (1H, dd, *J* = 7.7, 2.8 Hz, H-15), 4.13 (1H, dddd, *J* = 6.7, 6.2, 5.6, 5.1 Hz, H-3), 3.78 (1H, ddd, *J* = 8.2, 6.5, 1.9 Hz, H-7), 3.18 (1H, d, *J* = 5.6 Hz, 3-OH), 3.06 (1H, dq, *J=* 8.2, 7.1 Hz, H-6), 2.98 (1H, dq, *J =* 6.2, 7.0 Hz, H-4), 2.71 (3H, s, H-21), 2.64 (1H, dd, *J =* 15.1, 6.7 Hz, H-2a), 2.54 (1H, dd, *J =* 15.1, 5.1 Hz, H-2b), 2.44 (2H, m, H-14), 2.22 (1H, dddd, *J =* 13.8, 7.0, 6.2, 2.9 Hz, H-11a), 2.10 (3H, d, *J* = 1.1 Hz, H-27), 2.09 (1H, d, *J=* 6.5 Hz, 7-OH), 1.88 (1H, dddd, *J* = 13.8, 10.9, 7.4, 2.9 Hz, H-11b), 1.65 (1H, m, H-8), 1.63 (1H, m, H-10a), 1.56 (1H, dddd, *J* = 12.7, 12.7, 3.9, 3.9 Hz, H-9a), 1.20 (3H, d, *J =* 7.1 Hz, H-24), 1.15 (3H, d, *J* = 7.0 Hz, H-23), 1.13 (1H, m, H-10b), 1.04 (1H, m, H-9b), 1.01 (3H, d, *J* = 7.0 Hz, H-25); ¹³C NMR, see Table 1; EIMS *m*/z 463 [M]⁺ (13), 290 (11), 190 (10), 168 (100), 164 (20), 157 (26), 139 (17); HREIMS *m*/*z* 463.2383 (calcd. for C₂₅H₃₇NO₅S, 463.2392).

**Tab 1.**

| Aktivität von Epothilonen und Verbindungen (1) bis (39) gegen gegen Maus-Fibroblasten (L929, IC50 /ng/ml/) | | | | | |
|---|---|---|---|---|---|
| Strukturtyp | Epothilone | | | | |
| | A_{Y} | B_{Y} | C_{Y} | D_{Y} | trans C_{Y} |
| Ausgangsepothilon | (**1**) 4 | (**2**) 1-2 | (**14**) 50-100 | (**15**) 20 | - |
| 21-Hydroxy (E&F) | (**3**) 10 | (**4**) 1.5 | - | - | - |
| Oxazoles (G&H) | (**10**) 6 | (**11**) 1 | (**12**) 120 | (**13**) 11 | - |
| (*R*)-4-Desmethyl (X₁) | (**5**) 20 | - | (**16**) 200 | (**17**) 20 | (**28**) 400 |
| (*S*)-4-Desmethyl (X₂) | (**6**) 7 | - | (**18**) 25-30 | (**19**) 12 | (**29**) 80 |
| 6-Desmethyl (X₃) | - | - | (**20**) 1500 | - | - |
| 8-Desmethyl (X₄) | - | - | (**21**) 800 | - | - |
| 8,9-Dehydro (X₅) | - | - | (**22**) 1500 | (**23**) 200 | - |
| 10,11-Dehydro (X₆) | - | - | (**24**) 120 | - | - |
| 14-Hydroxy (X₇) | - | - | (**25**) | - | - |
| 16-Desmethyl (X₈) | (**7**) 20 | - | (**26**) 250 | - | - |
| 27-Hydroxy (X₉) | (**8**) 100 | - | (**27**) 200 | - | - |
| 21-Methyl (X₁₀) | - | (9) 1.5 | - | - | - |
| Verbindung | - | - | (**36**) 180 | | |
| Verbindung | - | - | (**37**) 50 | - | - |
| Verbindung | - | - | (**38**) 2000 | (**39**) 500 | - |

## Patentansprüche

1. Epothilon der Formel 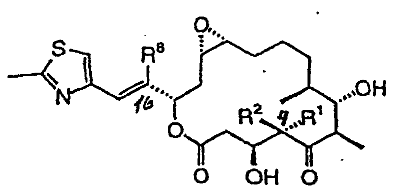
Epothilone A₁ (5) R¹ = H; R², R⁸ = Me oder
Epothilone A₂ (6) R² = H; R¹, R⁸ = Me oder
Epothilone A₈ (7) R⁸ = H; R¹, R² = Me

2. Epothilon der Formel 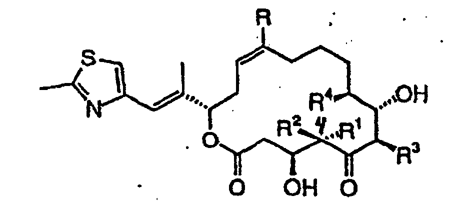
Epothilone C₁ (16) R¹ = H; R², R³, R⁴ = Me; R = H oder
Epothilone D₁ (17) R¹ = H; R², R³, R⁴ = Me; R = Me oder
Epothilone C₂ (18) R² = H; R¹, R³, R⁴ = Me; R = H oder
Epothilone D₂ (19) R² = H; R¹, R³, R⁴ = Me; R = Me

3. Epothilon der Formel 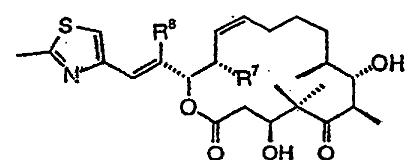
Epothilone C₈ (26) R⁸, R⁷ = H.

4. Epothilon der Formel 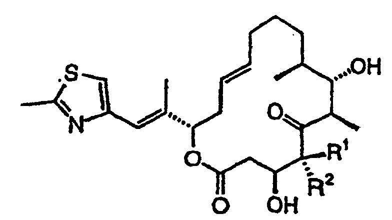
trans-Epothilone C₁ (28) R¹ = H; R² = Me oder
trans-Epothilone C₂ (29) R² = H; R¹ = Me

## Claims

1. Epothilone of the formula 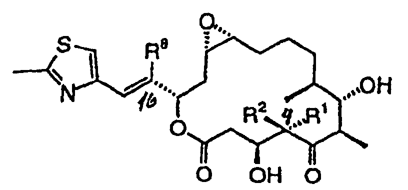
Epothilone A₁ (5) R¹ = H; R²,R⁸ = Me or
Epothilone A₂ (6) R² = H; R¹,R⁸ = Me or
Epothilone A₈ (7) R⁸ = H; R¹,R² = Me

2. Epothilone of the formula 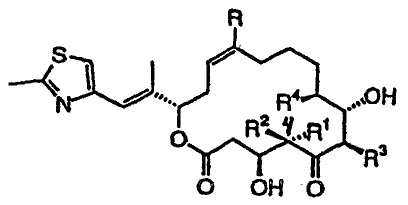
Epothilone C₁ (16) R¹ = H; R²,R³,R⁴ = Me; R = H or
Epothilone D₁ (17) R¹ = H; R²,R³,R⁴ = Me; R = Me or
Epothilone C₂ (18) R² = H; R¹,R³,R⁴ = Me; R = H or
Epothilone D2 (19) R² = H; R¹,R³,R⁴ = Me; R = Me

3. Epothilone of the formula 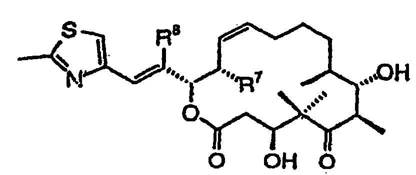
Epothilone C₈ (26) R⁸, R⁷ = H

4. Epothilone of the formula 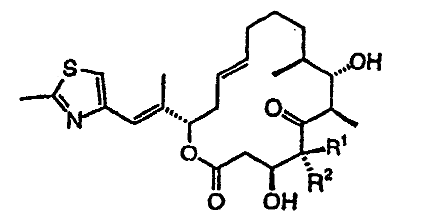
trans-Epothilone C₁ (28) R¹ = H; R² = Me or
trans-Epothilone C₂ (29) R² = H; R¹ = Me

## Revendications

1. Epothilone de la formule 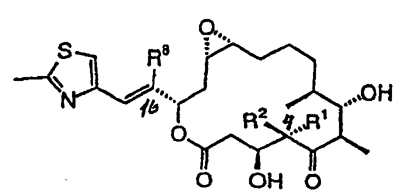
Epothilone A₁ (5) R¹ = H; R²,R⁸ = Me ou
Epothilone A₂ (6) R² = H; R¹,R⁸ = Me ou
Epothilone A₈ (7) R⁸ = H; R¹,R² = Me

2. Epothilone de la formule 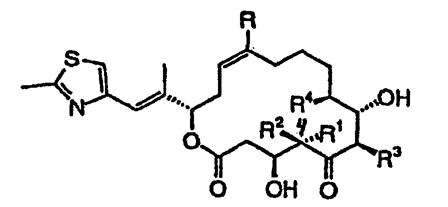
Epothilone C₁ (16) R¹ = H; R²,R³,R⁴ = Me; R = H ou
Epothilone D₁ (17) R¹ = H; R²,R³,R⁴ = Me; R = Me ou
Epothilone C₂ (18) R² = H; R¹,R³,R⁴ = Me; R = H ou
Epothilone D₂ (19) R² = H; R¹,R³,R⁴ = Me; R = Me

3. Epothilone de la formule 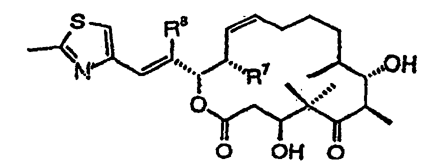
Epothilone C₈ (26) R⁸, R⁷ = H

4. Epothilone de la formule
trans-Epothilone C₁ (28) R¹ = H; R² = Me ou
trans-Epothilone C₂ (29) R² = H; R¹ = Me
